# EUROPEAN PATENT APPLICATION

(11) **EP 3 369 422 A1**
(43) Date of publication of application: **05.09.2018**
(21) Application number: 16859936.3
(22) Date of filing: 28.10.2016
(51) Int. Cl.: A61K 31/4535, A61K 9/08, A61K 31/4375, A61K 31/496, A61K 31/5383, A61K 31/7004, A61K 33/00, A61K 33/14, A61P 11/02, A61P 27/02, A61P 37/08, A61P 43/00

(54) **EXTERNAL PREPARATION**

(30) Priority: 29.10.2015 JP 2015213218
(71) Applicant: Teika Pharmaceutical Co., Ltd., Toyama-shi, Toyama 930-0982 (JP)
(72) Inventor: KIMURA, Takahito, Toyama-shi Toyama 930-0982 (JP); FUJISHITA, Shigeto, Toyama-shi Toyama 930-0982 (JP); SHIOTA, Satoshi, Toyama-shi Toyama 930-0982 (JP); ASO, Kenji, Toyama-shi Toyama 930-0982 (JP); OIDA, Hitoshi, Toyama-shi Toyama 930-0982 (JP); HORIUCHI, Isao, Toyama-shi Toyama 930-0982 (JP)
(74) Representative: Gulde & Partner
(86) International application number: PCT/JP2016/081990
(87) International publication number: WO 2017/073708

(57) **Abstract**

The present invention provides an external preparation that exerts excellent allergy treatment effects and comprises a combination of at least one ingredient (A) selected from the group consisting of ketotifen and pharmaceutically acceptable salts thereof; and at least one component (B) selected from the group consisting of quinolone antibacterial agents, saccharides, and inorganic salts.

## Description

### Technical Field

The present invention relates to external preparations.

### Background Art

Among various external ocular diseases, there is the biggest number of patients with allergic conjunctival disease having itching and/or hyperemia of eyes. In Japan alone, at least 20 million patients with allergic conjunctival disease are present, said a report.

This allergic conjunctival disease can be largely grouped into 5 disease subtypes: (1) seasonal allergic conjunctivitis; (2) perennial allergic conjunctivitis; (3) atopic conjunctivitis; (4) spring conjunctivitis; and (5) giant papillary conjunctivitis.
(1) Seasonal allergic conjunctivitis causes inflammation in the conjunctiva while plant pollens and others are main allergens. (2) Perennial allergic conjunctivitis causes inflammation in the conjunctiva while mites, house dust, and others are main allergens. In addition, (3) atopic conjunctivitis is a kind of allergic conjunctival disease and is accompanied by atopic dermatitis. This atopic conjunctivitis not only affects the surface of an eye(s) but also have a complication such as atopic cataract with lens opacity, a retinal tear(s) frequently observed at or near the ocular fundus, or rhegmatogenous retinal detachment. In some cases, the atopic conjunctivitis is known to be a disease at risk of ablepsia. (4) Spring conjunctivitis causes, in addition to symptoms of allergic conjunctivitis (1) and (2), a swelling called a papilla on the superior palpebral conjunctiva. In severe cases, the spring conjunctivitis causes a giant papilla. In addition, redness occurs on the bulbar conjunctiva near a cornea. In severe cases, the cornea is injured and may become opaque. (5) Giant papillary conjunctivitis, in general, causes a swelling called a papilla on the superior palpebral conjunctiva. Contact lens contamination and/or lens-derived stimulation are frequently responsible for the disease.

Treatment of such diseases involves removal of allergens and dosing of various antiallergic eye drops, steroid eye drops, or immunosuppressive eye drops. The antiallergic eye drops may contain, for example, olopatadine hydrochloride (Patent Literature 1), levocabastine hydrochloride (Patent Literature 2), tranilast (Patent Literature 3), ketotifen fumarate (Patent Literature 4), or sodium cromoglicate. The steroid eye drops may contain, for example, betamethasone sodium phosphate or fluorometholone. The immunosuppressive eye drops may contain, for example, tacrolimus hydrate (Patent Literature 5).

Among them, the ketotifen fumarate-containing eye drops are widely used for treatment of allergic disease as a drug having both an antiallergic action due to suppression of release of chemical mediators (e.g., histamine, SRS-A) from mast cells, basophils, and/or neutrophils and an anti-histamine action due to specific HI-receptor antagonism.

### Citation List

### Patent Literature

[Patent Literature 1] Japanese Patent No. 3068858
[Patent Literature 2] Japanese Patent No. 1526629
[Patent Literature 3] Japanese Patent No. 2090161
[Patent Literature 4] Japanese Patent No. 1065005
[Patent Literature 5] Japanese Patent No. 2536248

### Summary of Invention

### Technical Problem

As described above, various therapeutic agents have been used so as to treat allergic disease. Unfortunately, such therapeutic agents still cannot cure (alleviate) or suppress the allergy to a satisfactory level, so that a more potent drug has been sought.

### Solution to Problem

The present inventors have conducted intensive research and, as a result, have found that regarding a combination with ketotifen, ketotifen fumarate-containing eye drops are combined with a specific component(s), which is not used in conventional allergic disease treatment, to enhance their effects. In this way, the present inventors have completed the present invention.

Specifically, the present invention includes the following items [1] to [16].
[1] An external preparation comprising a combination of at least one ingredient (A) selected from the group consisting of ketotifen and pharmaceutically acceptable salts thereof; and at least one component (B) selected from the group consisting of quinolone antibacterial agents, saccharides, and inorganic salts.
[2] The external preparation according to item [1], comprising the ingredient (A) and the component (B).
[3] The external preparation according to item [1] or [2], wherein the quinolone antibacterial agents are at least one selected from the group consisting of ofloxacin, gatifloxacin, tosufloxacin, and salts thereof.
[4] The external preparation according to any one of items [1] to [3], wherein the saccharides are glucose.
[5] The external preparation according to any one of items [1] to [4], wherein the inorganic salts are at least one component selected from the group consisting of metal halides, carbonates or bicarbonates, and sulfates.
[6] The external preparation according to any one of items [1] to [5], wherein the inorganic salts are at least one component selected from the group consisting of sodium chloride and sodium bicarbonate.
[7] The external preparation according to any one of items [1] to [6], which is an ophthalmic preparation or nasal drops.
[8] The external preparation according to any one of items [1] to [7] for treatment of allergic disease.
[9] The external preparation according to any one of items [1] to [8] for treatment of allergic disease involving a cornea or a conjunctiva.
[10] The external preparation according to any one of items [1] to [9], which is an external aqueous liquid preparation.
[11] A kit for the manufacture of an external preparation, comprising:
   a preparation (a) comprising at least one ingredient selected from the group consisting of ketotifen and pharmaceutically acceptable salts thereof; and
   a preparation (b) comprising at least one component selected from the group consisting of quinolone antibacterial agents, saccharides, and inorganic salts.
[12] The kit according to item [11] for treatment of allergic disease.
[13] The kit according to item [11] or [12], wherein the preparation (a) and the preparation (b) are mixed in an indicated tissue by continuous dosing to a subject animal.
[14] The kit according to any one of items [11] to [13], further comprising a label indicating that the preparation (a) and the preparation (b) are mixed in an indicated tissue by continuous dosing to a subject animal.
[15] An enhancer of an antiallergic action of at least one ingredient (A) selected from the group consisting of ketotifen and pharmacologically acceptable salts thereof, the enhancer comprising at least one component (B) selected from the group consisting of quinolone antibacterial agents, saccharides, and inorganic salts.
[16] Use of at least one component (B) selected from the group consisting of quinolone antibacterial agents, saccharides, and inorganic salts for the manufacture of an enhancer of an antiallergic action of at least one ingredient (A) selected from the group consisting of ketotifen and pharmacologically acceptable salts thereof.
   The present invention further includes the following items [17] to [21].
[17] At least one component (B) selected from the group consisting of quinolone antibacterial agents, saccharides, and inorganic salts, which is used for enhancing an antiallergic action of at least one ingredient (A) selected from the group consisting of ketotifen and pharmacologically acceptable salts thereof.
[18] A method for enhancing an antiallergic action of at least one ingredient (A) selected from the group consisting of ketotifen and pharmacologically acceptable salts thereof, the method comprising:
   administering, to a mammal including a human being, at least one component (B) selected from the group consisting of quinolone antibacterial agents, saccharides, and inorganic salts.
[19] Use of at least one component (B) selected from the group consisting of quinolone antibacterial agents, saccharides, and inorganic salts for enhancing an antiallergic action of at least one ingredient (A) selected from the group consisting of ketotifen and pharmacologically acceptable salts thereof.
[20] A method comprising dropping, to an eye of a mammal including a human being, at least one ingredient (A) selected from the group consisting of ketotifen and pharmaceutically acceptable salts thereof in combination with at least one component (B) selected from the group consisting of quinolone antibacterial agents, saccharides, and inorganic salts.
[21] The method according to item [20], which is an allergic disease treatment method.

### Advantages

The present invention can provide a ketotifen-containing novel external preparation and, in particular, an external preparation having an excellent antiallergic action. The present invention, in particular, can provide a therapeutic agent having a higher efficacy in treatment of allergy than ketotifen alone.

### Brief Description of Drawing(s)

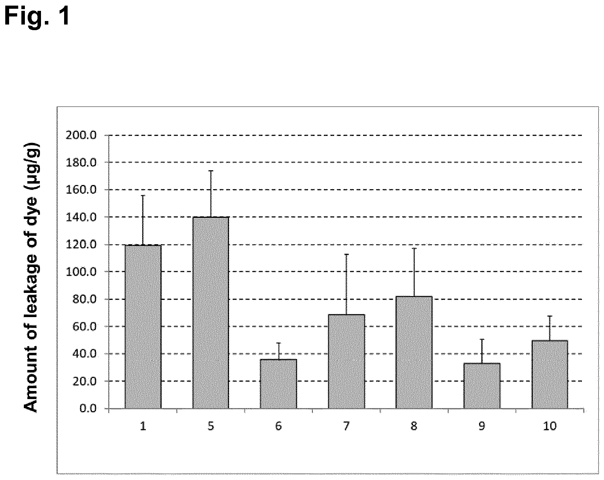

[FIG. 1] FIG. 1 is a graph showing an antiallergic effect of the present invention.

### Description of Embodiments

### [External Preparation]

An external preparation according to an embodiment of the present invention can be produced by combining: at least one ingredient (A) selected from the group consisting of ketotifen and pharmaceutically acceptable salts thereof; and a specific component(s) (B).

The ketotifen compound of the ingredient (A) is named 4,9-dihydro-4-(1-methyl-4-piperidylidene)-10H-benzo[4,5]-cyclohepta[1,2-b]thiphen-10-one. The ketotifen may be in a form of hydrate. The ketotifen and salts thereof are compounds known as antiallergic agents and may be synthesized by common procedures or may be commercially available ones.

As used herein, the pharmacologically acceptable salts of ketotifen of the ingredient (A) have no particular limitation as long as they are medically, pharmacologically, or physiologically acceptable. Specific examples include salts of the compound and an acid (e.g., an organic acid salt, an inorganic acid salt) and salts of the compound and a base (e.g., an organic base salt, an inorganic base salt). Usually used is an acid salt. Preferred is an organic acid salt. Examples of the organic acid salt include polycarboxylic acid salts (e.g., fumarate, maleate, succinate, malonate), monocarboxylic acid salts (e.g., trifluoroacetate, palmitate, stearate, acetate, butyrate), oxycarbonic acid salts (e.g., lactate, tartrate, citrate), and organic sulfonic acid salts (e.g., methanesulfonate, toluenesulfonate, tosylate). Preferred are polycarboxylic acid salts. More preferred are fumarate and maleate, etc. Still more preferred are fumarate, etc. Examples of the inorganic acid salt include salts of hydrochloric acid, sulfuric acid, nitric acid, phosphoric acid, hydrobromic acid, etc. Examples of the organic base salt include salts of organic amines (e.g., methylamine, triethylamine, triethanolamine, morpholine, piperazine, pyrrolidine, tripyridine, picoline). Examples of the inorganic base salt include ammonium salts and salts of metals (e.g., alkali metals (e.g., sodium, potassium), alkali earth metals (e.g., calcium, magnesium), aluminum).

The ingredient (A) of an external preparation of the present invention may be at least one selected from ketotifen and pharmacologically acceptable salts thereof. They may be used singly or in combination. The ingredient (A) used herein is preferably pharmacologically acceptable salts of ketotifen, more preferably a salt of ketotifen and an organic or inorganic acid, still more preferably a salt of ketotifen and an organic acid, still more preferably a salt of ketotifen and a polycarboxylic acid, still more preferably a salt of ketotifen and a fumaric acid or a maleic acid, and still more preferably a salt of ketotifen and a fumaric acid (i.e., ketotifen fumarate), etc.

The proportion of the ingredient (A) in an external preparation of the present invention is suitably determined depending on the kind of the ingredient (A), the kinds of the other components, etc. For instance, the proportion of the ingredient (A) to the total amount of the external preparation is from 0.01 to 1% (w/v), preferably from 0.03 to 0.5% (w/v), more preferably from 0.03 to 0.1% (w/v), and still more preferably from 0.04 to 0.06% (w/v). Depending on a form of the external preparation, the proportion of the ingredient (A) to the total amount of the external preparation may be from 0.05 to 1.6 mass%, preferably from 0.3 to 1.5 mass%, more preferably from 0.5 to 1.5 mass%, and still more preferably from 1 to 1.5 mass%. Note that the above numerical ranges may be represented in terms of ketotifen.

Examples of the component (B) combined with the ingredient (A) include quinolone antibacterial agents, saccharides, inorganic salts, organic acid salts, gentamicin, and cefmenoxime. Preferred are quinolone antibacterial agents, saccharides, inorganic salts, etc.

As used herein, the quinolone antibacterial agents of the component (B) are synthetic antibacterial agents, the backbone of which is a nalidixic acid-based pyridonecarboxylic acid. A new quinolone is included. Examples of the quinolone antibacterial agents include, but are not particularly limited to, ofloxacin, gatifloxacin, tosufloxacin, norfloxacin, moxifloxacin, enoxacin, ciprofloxacin, levofloxacin sparfloxacin, grepafloxacin, nadifloxacin, nalidixic acid, pipemidic acid, piromidic acid, lomefloxacin, fleroxacin, prulifloxacin, pazufloxacin, and linezolid. Preferred are ofloxacin, gatifloxacin, tosufloxacin, and salts thereof.

Examples of the salts include the above-described salts. Specific examples include tosyl acid salts (e.g., tosufloxacin tosylate), hydrochlorides (e.g., ciprofloxacin hydrochloride, lomefloxacin hydrochloride), and mesylic acid salts (e.g., pazufloxacin mesylate).

Note that these compounds and salts thereof may be in a form of hydrate (e.g., gatifloxacin hydrate, tosufloxacin tosylate hydrate, pipemidic acid trihydrate).

The quinolone antibacterial agents are known compounds and may be synthesized by common procedures or may be commercially available ones. They may be used singly or in combination.

Examples of the saccharides in the component (B) include, but are not particularly limited to, monosaccharides, disaccharides, and polysaccharides. Examples of the monosaccharides include pentoses (e.g., xylose, arabinose, ribose) and hexoses (e.g., glucose, fructose, lactose). Examples of the disaccharides include sucrose, maltose, and trehalose. Examples of the polysaccharides include dextrin. The saccharides of the component (B) are known compounds and may be synthesized by common procedures or may be commercially available ones. They may be used singly or in combination.

Examples of the inorganic salts of the component (B) include, but are not particularly limited to, inorganic acid salts and inorganic base salts. Examples of the inorganic acids used to form salts include hydrochloric acid, sulfuric acid, nitric acid, phosphoric acid, hydrobromic acid, carbonic acid, and bicarbonic acid. Examples of the inorganic bases used to form salts include ammonia and metals (e.g., alkali metals (e.g., sodium, potassium), alkali earth metals (e.g., calcium, magnesium)).

Note that the inorganic salts may be in a form of hydrate.

Specific examples of the inorganic salts include metal salts (e.g., halides such as metal halides (e.g., alkali or alkali earth metal halides such as sodium chloride, potassium chloride, calcium chloride, calcium chloride dihydrate)), carbonates or bicarbonates (e.g., metal carbonates or bicarbonates (e.g., alkali or alkaline-earth metal salts such as sodium bicarbonate)), and sulfates (e.g., metal sulfates (e.g., alkali or alkaline-earth metal salts such as magnesium sulfate, magnesium sulfate heptahydrate)). Preferred are sodium chloride and sodium bicarbonate, etc. They may be synthesized by common procedures or may be commercially available ones. They may be used singly or in combination.

Specific examples of the organic acid salts of the component (B) include citrates (e.g., sodium citrate), acetates (e.g., sodium acetate, potassium acetate, magnesium acetate), and hydrates thereof (e.g., trisodium citrate dihydrate, sodium acetate trihydrate, magnesium acetate tetrahydrate).

At least one component is selected and used as the component (B) in an external preparation of the present invention. They may be used singly or in combination.

Preferable examples of the component (B) used herein include ofloxacin, gatifloxacin, tosufloxacin tosylate, glucose, sodium chloride, sodium bicarbonate, and hydrates thereof (e.g., gatifloxacin hydrate, tosufloxacin tosylate hydrate). They may be used singly or in combination. Note that examples of a commercially available medicine containing glucose, sodium chloride, and sodium bicarbonate include OPEGUARD®-MA intraocular irrigating solution (20 mL).

The proportion of the component (B) in an external preparation of the present invention is suitably determined depending on the kind of the component (B) and the kinds of the other components. For instance, the proportion of the component (B) to the total amount of the external preparation is from 0.05 to 1.6% (w/v), preferably from 0.3 to 1.5% (w/v), more preferably from 0.5 to 1.5% (w/v), and still more preferably from 1 to 1.5% (w/v). Depending on a form of the external preparation, the proportion of the component (B) to the total amount of the external preparation is from 0.05 to 1.6 mass%, preferably from 0.3 to 1.5 mass%, more preferably from 0.5 to 1.5 mass%, and still more preferably from 1 to 1.5 mass%.

As used herein, the mass ratio (A)/(B) is suitably determined and may be, for example, from 1/0.01 to 1/100. The mass ratio (A)/(B) is preferably from 1/0.5 to 1/15, more preferably from 1/1 to 1/15, still more preferably from 1/2 to 1/15, still more preferably from 1/2 to 1/14, and still more preferably from 1/3 to 1/6 or from 1/13 to 1/15. Note that the above numerical ranges of the ingredient (A) may be represented in terms of ketotifen.

An external preparation according to the present invention may have various dosage forms depending on intended usage. An external preparation according to the present invention may be used as a formulation such as a pharmaceutical preparation or a quasi-drug. For instance, the usage may involve various applications such as ophthalmic preparations, nasal preparations, ear drop preparations, skin external preparations. Here, examples of the ophthalmic preparations include eye drops, synthetic lacrimal fluid, eye washes, eye ointment, contact lens mounting liquid, and contact lens care solutions (e.g., a contact lens disinfectant, contact lens preserving agent, contact lens cleaning preparation, contact lens washing and storing solution). In addition, examples of the nasal preparations include nasal drops and nasal washes. Examples of the ear drop preparations include ear drops. Preferable examples of an external preparation of the present invention include ophthalmic preparations. Particularly preferred are eye drops.

An external preparation according to the present invention may have various pH values depending on its purpose. The pH is preferably from 3 to 12, more preferably from 4 to 11, still more preferably from 5 to 10, still more preferably from 5 to 9, and still more preferably from 5 to 8.

When an external preparation of the present invention is an ophthalmic preparation or nasal drops, the dosage and the number of dosings may be suitably selected depending on, for example, the symptoms of a subject to be dosed. For instance, each adult may receive 1 to 3 drops per dosing about 1 to 6 times a day. When an external preparation of the present invention is a skin external preparation, the application amount and the number of applications may be suitably selected depending on, for example, the symptoms of a subject to be dosed. For instance, the ointment may be squeezed from a tube in an amount that covers the end to the first joint of a pointing finger and this ointment should be applied to each adult 1 to 6 times a day.

The daily adult dose of the ingredient (A) in an external preparation of the present invention may be, for example, from 0.02 to 1.0 mg/kg in terms of ketotifen. As used herein, the daily adult dose of the component (B) may be, for example, from 0.04 to 20.0 mg/kg.

An external preparation of the present invention is effective in preventing and treating (alleviating) allergic disease and may be used as a prophylaxis and a therapeutic agent for inflammatory disease. Examples of the allergic disease of interest include corneal and/or conjunctival allergic diseases. Examples of the allergic conjunctival disease include seasonal allergic conjunctivitis, perennial allergic conjunctivitis, atopic conjunctivitis, spring conjunctivitis, and giant papillary conjunctivitis. Examples of the inflammatory disease of interest include inflammatory eye diseases (e.g., blepharitis, conjunctivitis, keratitis, scleritis, episcleritis, anterior segment uveitis, postoperative inflammation).

Examples of a dosage form of an external preparation of the present invention include liquids and semi-solids (e.g., ointment). The liquids may be aqueous liquids.

### <Other Components>

An external preparation of the present invention is suitably combined with a preparation carrier, if necessary. Examples of the preparation carrier include solvents, dissolution aids, emulsifiers, tonicity agents, buffers, and pH modifiers. Further, if necessary, any know additives and pharmaceutically acceptable additives commonly used in the field of pharmaceutical agents (e.g., stabilizers, preservatives, antioxidants) may be used.

Examples of the solvents include, but are not particularly limited to, purified water, ethanol, propylene glycol, polyethylene glycol, macrogol, and edible oils (e.g., sesame oil, corn oil, olive oil). Examples of the dissolution aids include, but are not particularly limited to, propylene glycol, D-mannitol, benzyl benzoate, ethanol, triethanolamine, sodium carbonate, and sodium citrate.

Examples of the emulsifiers include, but are not particularly limited to, carmellose, hydroxypropylcellulose, propylene glycol, polyvinylpyrrolidone, methylcellulose, glycerin monostearate, polyvinyl alcohol, lecithin (egg yolk lecithin, soybean lecithin), deoxycholic acids, polyoxyethylene castor oil, polyoxyethylene hydrogenated castor oils, polyoxyethylene polyoxypropylene glycol, polyoxyethylene sorbitan monooleate (polysorbate 80), and monolaurate polyoxyethylene sorbitan. Preferred is lecithin. Egg yolk lecithin is more preferable. The content of the emulsifier may be from 0.1 to 3.0 mass% relative to the total mass of the external preparation.

Examples of the tonicity agents include, but are not particularly limited to, glucose, D-sorbitol, sodium chloride, D-mannitol, and glycerin. Preferred is glycerin. The content of the glycerin is determined by calculating an amount that makes the whole external preparation isotonic.

Examples of the buffers include, but are not particularly limited to, citrates (e.g., trisodium citrate dihydrate, sodium citrate, disodium citrate), phosphates (e.g., sodium dihydrogenphosphate, sodium phosphate, disodium hydrogenphosphate, potassium phosphate, dipotassium hydrogenphosphate), borates (e.g., sodium borate, potassium borate), acetates (e.g., sodium acetate trihydrate, sodium acetate, potassium acetate), and carbonates (e.g., sodium carbonate, sodium bicarbonate).

Examples of the pH modifiers include, but are not particularly limited to, hydrochloric acid and sodium hydroxide.

Examples of the stabilizers include, but are not particularly limited to, EDTA, sodium oleate, casein, and casein sodium salts. Examples of EDTA include EDTA-2Na and EDTA-4Na. The content of EDTA may be from 0.001 to 0.1 mass% relative to the total mass of the external preparation.

Examples of the preservatives include, but are not particularly limited to, quaternary ammonium salts (e.g., benzalkonium chloride, benzethonium chloride), paraoxybenzoates (e.g., ethyl parahydroxybenzoate, methyl parahydroxybenzoate, propyl parahydroxybenzoate, and butyl parahydroxybenzoate), chlorobutanol, benzyl alcohol, sodium dehydroacetate, and sorbic acid. Examples of an oxidation inhibitor include, but are not particularly limited to, sodium sulfite and ascorbic acid.

Examples of the antioxidants include polyphenols, ascorbic acid, t-butylhydroquinone, butylhydroxyanisole, butyl hydroxy toluene, L-cysteine hydrochloride, sodium hydrogen sulfite, α-tocopherol, and derivatives thereof.

An external preparation of the present invention is suitably combined with another active ingredient, if necessary. Examples of the above active ingredient include antiallergic agents other than ketotifen and pharmacologically acceptable salts thereof and drugs other than steroids.

The preparation carriers, additives, and other active ingredients of an external preparation of the present invention may be used singly or in combination depending on the dosage form of the preparation of interest. They may be commercially available ones. When the preparation carriers, additives, and other active ingredients are included, their content may be from 0.01 to 97.00 mass% relative to the total mass of the preparation and may be from 10 to 80 mass%. If the content is within the above ranges, these components usually exert sufficient effects and do not result in a loss of the effects of the present invention. The above preparation carriers, additives, and other active ingredients may be used as the component (B) of the present invention.

### (How to Prepare)

An external preparation of the present invention may be prepared by optionally adding, to desired amounts of the above ingredient (A) and component (B), other preparation components at desired concentrations and by adjusting the pH to within a suitable range.

As used herein, the term "comprising a combination" encompasses an embodiment "comprising" and an embodiment "combining". As used herein, if the above ingredient (A) and component (B) are combined, their dosage regimens include: simultaneous dosing of the (A) and (B) by independently using the same route or different routes; and sequential dosing of the (A) and (B) by independently using the same route or different routes.

If the above (A) and (B) of an external preparation of the present invention are combined, the timing of using the (A) and (B) is not particularly limited. The ingredient (A) may be subject to pre-dosing, simultaneous dosing, or post-dosing with respect to the component (B). Also, any combination of the dosings is acceptable. From the viewpoint of there being no pre-treatment burden on a subject, the simultaneous dosing of the (A) and (B) is preferable. As used herein, the term "pre-dosing" means that the ingredient (A) is dosed for a certain period to a subject who has not received the component (B). In addition, the term "simultaneous dosing" means that the ingredient (A) is administered, to a subject, continuously from the start to the end of dosing of (B) or for a certain period during the dosing of (B). The term "post-dosing" means that the ingredient (A) is administered for a certain period after the end of dosing of the component (B). Note that the interval between the dosings of (A) and (B) has no particular limitation. For instance, the interval may be 60 min or less, preferably 30 min or less, and more preferably 15 min or less. In addition, the interval between the dosings of (A) and (B) has no particular limitation and is preferably 2 min or more and more preferably 5 min or more.

If an external preparation of the present invention contains the ingredient (A) and the component (B), the (A) and (B) are pre-mixed to produce the external preparation.

As used herein, the daily adult dose of the ingredient (A) may be, for example, from 0.02 to 1.0 mg/kg in terms of ketotifen. As used herein, the daily adult dose of the component (B) may be, for example, from 0.04 to 20.0 mg/kg.

### (Advantages)

The present invention can provide an external preparation, namely a therapeutic agent having a higher efficacy in treatment of allergy. This external preparation exerts better antiallergic effects (e.g., prevention, treatment, and alleviation of allergic diseases) than the ingredient (A) alone while the ingredient (A) and the component (B), for example, are combined. The advantageous effects of this external preparation can be demonstrated such that in a tissue of an animal that is treated with, for instance, ovalbumin (OBA) to trigger an allergic reaction, an amount of leakage of dye Evans blue is measured (by reading absorbance at 620 nm) to estimate how much vascular hyperpermeability is suppressed and the results are then compared with those of the ingredient (A) alone.

### [Kit]

An embodiment of the present invention provides a kit for producing an external preparation, the kit comprising: a preparation (a) comprising at least one ingredient selected from the group consisting of ketotifen and pharmaceutically acceptable salts thereof; and a preparation (b) comprising at least one component selected from the group consisting of quinolone antibacterial agents, saccharides, and inorganic salts.

The preparation (a) of this kit is described in the same manner as for the ingredient (A) of an external preparation of the present invention. The preparation (b) of this kit is described in the same manner as for the component (B) of an external preparation of the present invention. Agents contained in this kit include a pharmaceutical agent, a quasi-drug, and a cosmetic, etc. Examples of the pharmaceutical agent include pharmaceutical compositions.

The preparation (a) may include components (e.g., a preparation carrier, additive, another active ingredient) other than the ingredient in the preparation (a). The components other than the ingredient in the preparation (a) are described in the same manner as for the preparation carriers, additives, and other active ingredients of an external preparation of the present invention. The preparation (b) may include additional components (e.g., a preparation carrier, additive, another active ingredient) other than the components in the preparation (b). The components other than the components in the preparation (b) are described in the same manner as for the preparation carriers, additives, and other active ingredients of an external preparation of the present invention.

This kit may be provided as a kit desirably comprising a container (e.g., an eye drops container, a nasal drops container, an ointment case) containing the preparation (a) and the preparation (b).

This kit may be used while providing separate containers (e.g., an eye drops container, a nasal drops container, an ointment case), each including the preparation (a) or the preparation (b). This kit form is very useful if different components are administered with different dosing intervals or if individual components mixed by a physician are applied.

The preparation (a) and the preparation (b) may be mixed in an indicated tissue by continuous dosing to a subject animal. Examples of the subject animal include mammals including a human being. The wording "continuous dosing" means that after the start of dosing of the preparation (a), the preparation (b) is administered to a subject animal for a certain period or after the start of dosing of the preparation (b), the preparation (a) is administered to a subject animal for a certain period. There may or may not be an overlapping period between the dosings of the (a) and (b). If there is no overlapping period, an interval between the dosings of the (a) and (b) may be present. The interval has no particular limitation and may be, for example, 60 min or less, preferably 30 min or less, and more preferably 15 min or less. In addition, the interval has no particular limitation and is preferably 2 min or more and more preferably 5 min or more.

Examples of the indicated tissue include an ocular-mucous membrane, a nasal cavity, and a skin. An embodiment of this kit involves mixing the preparation (a) and the preparation (b) in an indicated tissue even if the (a) and (b) are administered with an interval. This kit may have a label indicating that the preparation (a) and the preparation (b) may be mixed in an indicated tissue by continuous dosing to a subject animal.

This kit is effective in preventing and treating (alleviating) allergic disease and may be used as a prophylaxis and a therapeutic agent for inflammatory disease.

### [Combination Product and Enhancer]

The present invention provides a preparation for the manufacture of a combination product containing the component (B) in combination with the ingredient (A). The combination product may exert antiallergic effects. The antiallergic effects involve prevention, treatment, and alleviation of allergic diseases. The combined component (B) frequently helps augment the antiallergic effects more strongly than the ingredient (A) alone. Because of this, this preparation may help augment or increase the antiallergic effects of the ingredient (A) (i.e., an antiallergic effect enhancer). Note that the antiallergic effects of this combination product can be demonstrated such that in a tissue of an animal in which an allergic reaction is triggered, an amount of leakage of dye Evans blue is measured (by reading absorbance at 620 nm). How much the antiallergic effects of the ingredient (A) are enhanced can be demonstrated such that in a tissue of an animal in which an allergic reaction, for example, is triggered, an amount of leakage of dye Evans blue is measured (by reading absorbance at 620 nm) to estimate how much vascular hyperpermeability is suppressed and the results are then compared with those of the ingredient (A) alone.

### [Usage]

The present invention provides use of a preparation (in particular, an enhancer of an antiallergic action of the ingredient (A)) for the manufacture of a combination product containing the component (B) in combination with the ingredient (A).

The present invention provides use of the component (B) for the manufacture of a combination product in combination with the ingredient (A).

### [Eye Drop Instillation]

An embodiment of the present invention provides a method for eye drop instillation, comprising dropping a combination of the ingredient (A) and the component (B) to an eye of a mammal including a human being. As used herein, the wording "dropping a combination to an eye" encompasses an embodiment "dropping a composition comprising the ingredient (A) and the component (B) to an eye" and an embodiment "dropping each of separate (A) and (B) to an eye while the respective (A) and (B) are combined on the eye". This method for eye drop instillation may involve an allergic disease treatment method (i.e., a method for preventing and treating (alleviating) allergic disease) and a method for preventing and treating inflammatory disease.

### [Other Matters]

An embodiment of the present invention includes the component (B) used to augment the antiallergic effects of the ingredient (A).

An embodiment of the present invention provides a method for enhancing antiallergic effects of the ingredient (A), comprising administering the component (B) to a mammal including a human being.

An embodiment of the present invention provides an external preparation that comprises the ingredient (A) and comes with a package insert where it is recommended that the ingredient (A) and the component (B) are simultaneously or continuously administered.

An embodiment of the present invention provides an external preparation that comprises the component (B) and comes with a package insert where it is recommended that the ingredient (A) and the component (B) are simultaneously or continuously administered.

An embodiment of the present invention provides an external preparation that comprises the ingredient (A) and comes with a package insert indicating that the ingredient (A) and the component (B) are simultaneously or continuously administered so as to augment their effects.

An embodiment of the present invention provides an external preparation that comprises the component (B) and comes with a package insert indicating that the ingredient (A) and the component (B) are simultaneously or continuously administered so as to augment their effects.

### Examples

The present invention is further described in detail by referring to the following Examples. However, they do not restrict the present invention.

### [Example 1: Case where the component (B) is a quinolone antibacterial agent]

### (How to Prepare)

As test solutions were prepared the following four groups:
1. a control group (physiological saline);
2. a group of ingredient (A) alone (Zaditen® eye drops 0.05% (w/v); manufactured by Alcon Japan Ltd.);
3. a group of component (B) alone (pharmaceutical eye drop agents); and
4. a group of combination product (in which ketotifen is dissolved or suspended at 0.05% (w/v) in pharmaceutical eye drop agents).

As a trigger solution was used a solution prepared by mixing equal volumes of 1% (w/v) ovalbumin (Grade V; manufactured by Sigma-Aldrich Co. LLC)/physiological saline (manufactured by Otsuka Pharmaceutical Co., Ltd.) and 1% (w/v) Evans blue (manufactured by Wako Pure Chemical Industries, Ltd.)/physiological saline.

As an extraction solution was used an aqueous solution of acetone (manufactured by Wako Pure Chemical Industries, Ltd.)/0.5% (w/v) sodium sulfate (manufactured by Wako Pure Chemical Industries, Ltd.) (7:3).

### (Test)

Rats (male, 5-week-old (at the time of transport), Wistar rats; purchased from Japan SLC, Inc.) were subjected to diethyl ether inhalation anesthesia; and a pre-made anti-ovalbumin antiserum (antibody titer: 5) was then injected under both inferior palpebral conjuctivas to induce passive immunization (20 µL/eye; n = 3/group). After 48 h, 1 mL of the trigger solution was intravenously administered to trigger an allergic reaction at the conjunctiva sites. Then, each test solution was dropped onto the eyes at 15 min before and immediately before triggering the allergic reaction (the total of 2 times). At 30 min after triggering the reaction, each rat was euthanized; the inferior palpebral conjuctivas were resected along the conjunctival fornix; the tissues were weighed; the extraction solution was then added; and extraction was carried out overnight. The overnight extraction solution was centrifuged to recover a supernatant. Absorbance of the supernatant was measured at 620 nm.

### (Evaluation Procedure)

Vascular hyperpermeability caused by an allergic reaction results in leakage of Evans blue. Thus, the less the amount of leakage of the dye, the more the vascular hyperpermeability is suppressed. This should be an indicator of the antiallergic effects. The amount of leakage of dye per tissue unit (µg/g) was calculated by using a predetermined standard curve. A dye amount ratio was calculated using the following equation to give an indicator of the suppression effects.

Dye amount ratio = (Average dye amount (a group of combination product)) / (Average dye amount (a control group, a group of ingredient (A) alone, or a group of component (B) alone).

### (Results)

Tables 1 shows the results. In Table 1, the symbol "%" means "% (w/v)". Because the dye amount ratio (the group of combination product / the control group) is less than 1, the group of combination product evidently exerts antiallergic effects. Surprisingly, the dye amount ratio (the group of combination product / the group of ingredient (A) alone or the group of component (B) alone) was less than 1. This demonstrated that when the ingredient (A) and a quinolone antibacterial agent, which was ofloxacin, gatifloxacin, or tosufloxacin tosylate hydrate, were combined, the antiallergic effects were larger than those of the ingredient (A) or component (B) alone. Note that because the group of combination product containing the ingredient (A) and oxybuprocaine hydrochloride, bimatoprost, or diquafosol sodium had a dye amount ratio (the group of combination product / the control group) of less than 1, this group was demonstrated to exert the antiallergic effects.

**[Table 1]**

| Each pharmaceutical eye drop preparation (trade name) | Dye amount ratio (Group of combination product /) | | |
|---|---|---|---|
| | Control group | Group of ingredient (A) alone | Group of component (B) alone |
| Ofloxacin (Tarivid® eye drops 0.3%) | 0.47 | 0.62 | 0.57 |
| Gatifloxacin (Gatiflo® eye drops 0.3%) | 0.43 | 0.64 | 0.45 |
| Tosufloxacin tosylate hydrate (Ozex® eye drops 0.3%) | 0.50 | 0.69 | 0.67 |
| Oxybuprocaine hydrochloride (Lacrimin® eye drops 0.05%) | 0.76 | 2,29 | - |
| Bimatoprost (Lumigan® eye drops 0.03%) | 0.70 | 1.69 | - |
| Diquafosol sodium (Diquas® eye drops 0.3%) | 0.82 | 1.49 | - |

### [Example 2: Case where the Component (B) is saccharides or inorganic salts]

As test solutions were prepared the following 6 groups listed in Table 2. The same trigger solution, extraction solution, and control group as of Example 1 were used and the same test as of Example 1 was carried out except that the number n of rats in each group was 4. Note that 0.069% (w/v) of ketotifen fumarate in Table 2 is equal to 0.05% (w/v) in terms of ketotifen.

**[Table 2]**

| Unit: % (w/v) | | | | | | |
|---|---|---|---|---|---|---|
| | 5 (Group of component (B) alone) | 6 | 7 | 8 | 9 | 10 |
| Ketotifen fumarate | - | 0.069 | 0.069 | 0.069 | 0.069 | 0.069 |
| Glucose | 0.150 | 0.150 | - | 0.150 | 0.150 | 0.150 |
| Sodium chloride | 0.660 | 0.660 | 0.660 | - | 0.660 | 0.660 |
| Potassium chloride | 0.036 | 0.036 | 0.036 | 0.036 | - | 0.036 |
| Calcium chloride dihydrate | 0.018 | 0.018 | 0.018 | 0.018 | 0.018 | 0.018 |
| Magnesium sulfate heptahydrate | 0.030 | 0.030 | 0.030 | 0.030 | 0.030 | 0.030 |
| Sodium bicarbonate | 0.210 | 0.210 | 0.210 | 0.210 | 0.210 | - |
| Trisodium citrate dihydrate | 0.124 | 0.124 | 0.124 | 0.124 | 0.124 | 0.124 |
| Sodium acetate trihydrate | 0.060 | 0.060 | 0.060 | 0.060 | 0.060 | 0.060 |
| Hydrochloric acid | Suitable amount | Suitable amount | Suitable amount | Suitable amount | Suitable amount | Suitable amount |
| Purified water | Suitable amount | Suitable amount | Suitable amount | Suitable amount | Suitable amount | Suitable amount |

### (Evaluation Procedure)

The amounts of leakage of dye were compared with respect to the presence or absence of glucose, sodium chloride, potassium chloride, or sodium bicarbonate, etc.

### (Results)

FIG. 1 shows the results. The result of group 1 is compared with each of the results of groups 6 to 10. The comparison has demonstrated excellent antiallergic effects exerted by the groups (groups 6 to 10) of combination product containing ketotifen fumarate and saccharides (glucose), inorganic salts (sodium chloride, potassium chloride, calcium chloride dihydrate, magnesium sulfate heptahydrate, sodium bicarbonate), and organic salts (trisodium citrate dihydrate, sodium acetate trihydrate), etc. In addition, the result of group 6 is compared with the result of group 7, 8, or 10. This comparison has demonstrated that each of glucose, sodium chloride, and sodium bicarbonate can contribute to an antiallergic action when combined with ketotifen fumarate.

### Industrial Applicability

The present invention is useful in the field of medicine such as treatment of allergy.

## Claims

1. An external preparation comprising a combination of at least one ingredient (A) selected from the group consisting of ketotifen and pharmaceutically acceptable salts thereof; and at least one component (B) selected from the group consisting of quinolone antibacterial agents, saccharides, and inorganic salts.

2. The external preparation according to claim 1, comprising the ingredient (A) and the component (B).

3. The external preparation according to claim 1 or 2, wherein the quinolone antibacterial agents are at least one selected from the group consisting of ofloxacin, gatifloxacin, tosufloxacin, and salts thereof.

4. The external preparation according to any one of claims 1 to 3, wherein the saccharides are glucose.

5. The external preparation according to any one of claims 1 to 4, wherein the inorganic salts are at least one component selected from the group consisting of metal halides, carbonates or bicarbonates, and sulfates.

6. The external preparation according to any one of claims 1 to 5, wherein the inorganic salts are at least one component selected from the group consisting of sodium chloride and sodium bicarbonate.

7. The external preparation according to any one of claims 1 to 6, which is an ophthalmic preparation or nasal drops.

8. The external preparation according to any one of claims 1 to 7 for treatment of allergic disease.

9. The external preparation according to any one of claims 1 to 8 for treatment of allergic disease involving a cornea or a conjunctiva.

10. The external preparation according to any one of claims 1 to 9, which is an external aqueous liquid preparation.

11. A kit for the manufacture of an external preparation, comprising:
a preparation (a) comprising at least one ingredient selected from the group consisting of ketotifen and pharmaceutically acceptable salts thereof; and
a preparation (b) comprising at least one component selected from the group consisting of quinolone antibacterial agents, saccharides, and inorganic salts.

12. The kit according to claim 11 for treatment of allergic disease.

13. The kit according to claim 11 or 12, wherein the preparation (a) and the preparation (b) are mixed in an indicated tissue by continuous dosing to a subject animal.

14. The kit according to any one of claims 11 to 13, further comprising a label indicating that the preparation (a) and the preparation (b) are mixed in an indicated tissue by continuous dosing to a subject animal.

15. An enhancer of an antiallergic action of at least one ingredient (A) selected from the group consisting of ketotifen and pharmacologically acceptable salts thereof, the enhancer comprising at least one component (B) selected from the group consisting of quinolone antibacterial agents, saccharides, and inorganic salts.

16. Use of at least one component (B) selected from the group consisting of quinolone antibacterial agents, saccharides, and inorganic salts for the manufacture of an enhancer of an antiallergic action of at least one ingredient (A) selected from the group consisting of ketotifen and pharmacologically acceptable salts thereof.

17. At least one component (B) selected from the group consisting of quinolone antibacterial agents, saccharides, and inorganic salts, which is used for enhancing an antiallergic action of at least one ingredient (A) selected from the group consisting of ketotifen and pharmacologically acceptable salts thereof.

18. A method for enhancing an antiallergic action of at least one ingredient (A) selected from the group consisting of ketotifen and pharmacologically acceptable salts thereof, the method comprising:
administering, to a mammal including a human being, at least one component (B) selected from the group consisting of quinolone antibacterial agents, saccharides, and inorganic salts.

19. Use of at least one component (B) selected from the group consisting of quinolone antibacterial agents, saccharides, and inorganic salts for enhancing an antiallergic action of at least one ingredient (A) selected from the group consisting of ketotifen and pharmacologically acceptable salts thereof.

20. A method comprising dropping, to an eye of a mammal including a human being, at least one ingredient (A) selected from the group consisting of ketotifen and pharmaceutically acceptable salts thereof in combination with at least one component (B) selected from the group consisting of quinolone antibacterial agents, saccharides, and inorganic salts.

21. The method according to claim 20, which is an allergic disease treatment method.
